# EUROPÄISCHE PATENTSCHRIFT

(11) **EP 3 171 909 B1**
(45) Veröffentlichungstag und Bekanntmachung des Hinweises auf die Patenterteilung: **27.09.2023**
(21) Anmeldenummer: 15750924.1
(22) Anmeldetag: 23.07.2015
(51) Int. Cl.: A61M 1/14, A61M 1/34, A61M 1/36

(54) **DIALYSEGERÄT**
DIALYSIS APPARATUS
APPAREIL DE DIALYSE

(30) Priorität: 23.07.2014 DE 102014010906
(43) Veröffentlichungstag der Anmeldung: 31.05.2017
(73) Patentinhaber: Fresenius Medical Care Deutschland GmbH, 61352 Bad Homburg (DE)
(72) Erfinder: PETERS, Arne, 61352 Bad Homburg (DE)
(74) Vertreter: Herrmann, Uwe
(86) Internationale Anmeldenummer: PCT/EP2015/001527
(87) Internationale Veröffentlichungsnummer: WO 2016/012099

(56) Entgegenhaltungen:
- DE-A1-102007 042 964
- US-A1- 2002 198 483
- US-A1- 2005 038 325
- US-A1- 2008 041 792
- US-A1- 2009 124 963
- US-A1- 2011 315 611

## Beschreibung

Die vorliegende Erfindung betrifft ein Dialysegerät mit wenigstens einem Gehäuse und mit wenigstens einem Sensor, der wenigstens ein Sensorelement aufweist, das zur Erfassung von Feuchtigkeit an der Oberfläche des Gehäuses ausgebildet ist.

Bei der Behandlung von Patienten mit Dialysegeräten steht neben der Blutreinigung die zuverlässige Kontrolle des Wasserhaushaltes und des Entzuges von Wasser über die Membran des Dialysators im Vordergrund. Um die Behandlungsdauer in einem akzeptablen Rahmen zu halten, sind hohe Flüssigkeitsmengen und -raten wünschenswert. Um dabei die genaue Kontrolle über die zugeführten und abgezogenen Flüssigkeitsmengen durchführen zu können, bedarf es hochgenauer Flüssigkeitsbilanzsysteme.

Kommt es zu einer Leckage, führt dies neben der Kontamination der Oberfläche zu einem Bilanzfehler, der schnell und zuverlässig erkannt werden muss.

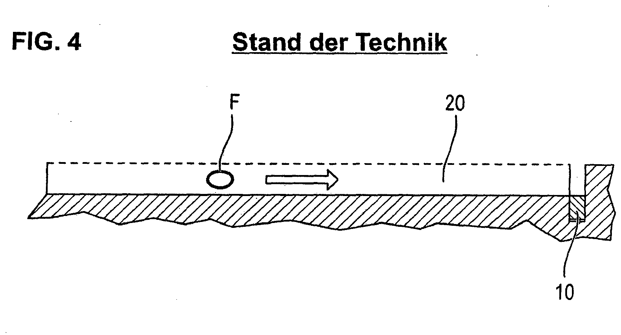

Aus dem Gebiet der Dialysegeräte sind Leckagesensoren bekannt. Aus Figur 4 ist beispielsweise ein Feuchtigkeitssensor 10 ersichtlich, der im Endbereich einer Rinne 20 angeordnet ist. Kommt es zu einer Leckage, läuft die Flüssigkeit F entlang der Rinne 20 zu dem Sensor 10 und kann dort erfasst und ggf. zur Erzeugung eines Alarmsignals verwendet werden. Figur 4 zeigt eine Schnittstelle eines Dialysegerätes zu einem Einmalartikel, d.h. zu einem Disposable. Die im Bereich der Schnittstelle austretende Flüssigkeit wird mittels der Rinne 20 zu dem Sensor 10 geführt.

Ein weiteres Einsatzgebiet von Feuchtigkeitssensoren bei Dialysegeräten sind Sensoren, die eine Diskonnektion des Patientenzugangs erfassen. Exemplarisch wird auf die US 2002/198483 A1 und auf die US 2005/038325 A1 verwiesen.

Bei dem aus Figur 4 bekannten Sensor besteht ein Nachteil darin, dass aufgrund des nur lokal angeordneten Sensors 10 eine Leckage zwischen der Oberfläche des Gerätes und der Schnittstelle nicht immer zuverlässig detektiert werden kann. Voraussetzung für die Erfassung von Feuchtigkeit bei dem aus Figur 4 bekannten System ist es, dass die Flüssigkeit zu dem Sensor hin geleitet wird.

Weiterhin ist aus der US2011315611 A1 eine tragbare Dialysemaschine mit einer abnehmbaren Steuereinheit und einer Basiseinheit bekannt.

Zudem offenbart die DE102007042964 A1 eine Vorrichtung zur Behandlung einer medizinischen Flüssigkeit, welche eine Behandlungsmaschine mit einer Ankoppelfläche umfasst, wobei eine Kassette aus einem Hartteil mit flüssigkeitsführenden Kanälen, welche von einer flexiblen Folie abgedeckt sind, an die Ankoppelfläche der Behandlungsmaschine ankoppelbar ist.

Darüberhinaus offenbart die US2002198483 A1 eine Vorrichtung zum Erfassen einer Verlagerung einer in einen Patienten eingeführten Nadel, umfassend einen Sensor zum Erfassen von Nässe aufgrund von Blut und einen Sensorhalter zum Befestigen des Sensors am Patienten, so dass der Sensor Nässe aufgrund eines Blutverlusts des Patienten erkennt.

Ähnlich dazu wird gemäß US2005038325 A1 mittels eines Sensors die Funktion einer Fistelnadel überwacht.

Auch die US2008041792 A1 betrifft ein Feuchtedetektorsystem, das das Vorhandensein von Feuchtigkeit aufgrund eines Flüssigkeitslecks in einem System schnell erfasst. US 2009/124963 A1 offenbart ein weitere Dialysemaschine nach dem Stand der Technik.

Der vorliegenden Erfindung liegt daher die Aufgabe zugrunde, ein Dialysegerät der eingangs genannten Art dahingehend weiterzubilden, dass die Zuverlässigkeit bei der Leckageerfassung gegenüber bekannten Anordnungen verbessert ist.

Diese Aufgabe wird durch ein Dialysegerät mit den Merkmalen des Anspruchs 1 gelöst.

Danach ist vorgesehen, dass sich das Sensorelement über einen flächigen Bereich der Oberfläche des Gehäuses erstreckt. Es findet somit keine nur lokale Erfassung von Feuchtigkeit statt, sondern eine flächige Detektion, was beispielsweise im Falle der Erfassung der Leckage zwischen einem Disposable und dem Dialysegerät den Vorteil mit sich bringt, dass Feuchtigkeit, die sich in dem engen Spalt zwischen Disposable und Dialysegerät befindet, mit größerer Zuverlässigkeit erfasst werden kann, als bei einer nur lokalen Anordnung eines Sensors.

Ein weiterer wesentlicher Vorteil der Erfindung besteht darin, dass die Erfassung des Vorhandenseins von Feuchtigkeit auch über gekrümmte Flächen, d.h. über eine dreidimensionale geometrische Form ohne Weiteres möglich ist.

Die vorliegende Erfindung betrifft des Weiteren ein Dialysegerät mit wenigstens einem Gehäuse und mit wenigstens einer an dem Gehäuse angeordneten Disposablekassette zur Führung wenigstens einer Flüssigkeit sowie mit wenigstens einem Sensor, der wenigstens ein Sensorelement aufweist, das zur Erfassung von Feuchtigkeit ausgebildet ist, wobei das Sensorelement an der Disposablekassette angeordnet ist und sich über einen flächigen Bereich der Oberfläche der Disposablekassette erstreckt.

Gemäß einer nicht beanspruchten Ausführungsform muss das Sensorelement somit nicht zwingend im Bereich der Oberfläche des Gehäuses des Dialysegerätes angeordnet sein. Alternativ oder zusätzlich dazu kann gemäß einer nicht beanspruchten Ausführungsform vorgesehen sein, dass sich das Sensorelement an der Disposablekassette befindet. Auch in diesem Fall ist vorgesehen, dass es sich nicht um ein lokales, d.h. nur punktuell vorhandenes Sensorelement handelt, sondern dass sich das Sensorelement über einen flächigen Bereich der Oberfläche der Disposablekassette erstreckt. Auch dadurch lässt sich beispielsweise Flüssigkeit bzw. Feuchtigkeit, die sich durch Leckage in dem engen Spalt zwischen der Disposablekassette und der Maschinenkonsole bzw. der Oberfläche des Dialysegerätes befindet, zuverlässig detektieren. Auch eine Erfassung in einer dreidimensional gekrümmten Fläche des Disposables ist auf diese Weise ohne Weiteres möglich.

In einer weiteren Ausgestaltung der Erfindung ist vorgesehen, dass das Dialysegerät keine Bohrung zur Aufnahme des Sensors oder des Sensorelementes und keine Rillen oder sonstige Bahnen zur Ableitung der Feuchtigkeit zu dem Sensor bzw. zu dem Sensorelement hin aufweist.

Im Vordergrund steht somit nicht eine bestimmte Ableitung der Flüssigkeit, sondern es wird erfindungsgemäß eine Oberfläche, nämlich die Oberfläche der Schnittstelle zu dem Disposable verwendet, die gleichzeitig das Gehäuse darstellt und als Sensor dient.

Grundsätzlich kann gemäß einer nicht beanspruchten Ausführungsform das Sensorelement an beliebigen freiliegenden Flächen des Dialysegerätes bzw. der Disposablekassette angeordnet sein.

Das Sensorelement kann derart ausgebildet sein, dass es zur Erfassung jeder beliebigen Flüssigkeit wie beispielsweise von Blut oder Dialyseflüssigkeit dient.

Der erfindungsgemäße Flächensensor ist wie oben ausgeführt ein Gehäuseteil.

Der Flächensensor kann gemäß einer nicht beanspruchten Ausführungsform ein Disposableteil bzw. Bestandteil von deren Oberfläche sein.

Gemäß der vorliegenden Erfindung ist das Sensorelement, d.h. der Bestandteil des Sensors, der die eigentliche Erfassung der Anwesenheit von Flüssigkeit vornimmt, in Form von einer oder mehreren Bahnen oder in Form wenigstens einer Matrix ausgebildet. Diese Bahnen bzw. diese Matrix kann sich entlang der Oberfläche des Gehäuses und/oder eines Disposables und vorzugsweise der Disposablekassette erstrecken.

Eine Disposablekassette dient beispielsweise der Förderung von Dialyseflüssigkeit und kann Anschlusselemente zur Konnektion von Lösungsbeuteln bzw. Anschlusselementen zum Anschluss an den Patienten bzw. an das Dialysegerät aufweisen. Des Weiteren kann ein solches Disposable Ventile oder dergleichen zum Steuern des Flüssigkeitsstromes aufweisen. Ein Beispiel für eine Disposablekassette ist aus der DE 198 14 695 C2 bekannt.

Gemäß der vorliegenden Erfindung ist vorgesehen, dass der Abstand zwischen benachbarten Bahnen oder benachbarten Elementen der genannten Matrix zueinander 1 cm oder weniger beträgt. Auf diese Weise ist eine zuverlässige Detektion von einzelnen Tropfen bzw. von Feuchtigkeit möglich.

In einer denkbaren Ausführungsform der Erfindung ist vorgesehen, dass das Sensorelement wenigstens eine elektrisch leitende Bahn oder wenigstens eine elektrisch leitende Sensormatrix umfasst und wobei der Sensor des Weiteren Mittel zur Erfassung der Änderung des Widerstandes oder der Leitfähigkeit der elektrisch leitenden Bahn oder der elektrisch leitfähigen Sensormatrix umfasst. Denkbar ist es beispielsweise, dass die elektrisch leitende Bahn oder die Sensormatrix durch ein Metall oder durch einen graphitgefüllten Kunststoff gebildet werden. Denkbar ist es somit beispielsweise, dass graphitgefüllte Kunststoffe in das Gehäuse des Dialysegerätes eingebracht werden und dort als Sensorfläche dienen.

Wird die Oberfläche mit einer oder mehreren Leiterbahnen durchsetzt, die voneinander beabstandet sind, überbrückt vorhandene Flüssigkeit wenigstens zwei benachbarte Leiterbahnen, wodurch sich der Widerstand ändert. Dies kann durch den Sensor bzw. eine Auswerteeinheit des Sensors ausgewertet werden und es kann auf eine Leckage rückgeschlossen werden. Eine derartige resistive Messung erfasst somit eine Widerstandsänderung zwischen den Leiterbahnen, bedingt durch einen Flüssigkeitstropfen.

Entsprechendes gilt für die Anordnung elektrisch leitfähiger Elemente in Form einer Matrix. Die einzelnen Elemente der Matrix können ebenfalls durch ein Metall oder auch durch einen elektrisch leitfähigen Kunststoff gebildet werden. Dabei kann die Anordnung der einzelnen Matrixelemente auf eine bestimmte Geometrie angepasst werden, nämlich dort wo insbesondere mit einer Leckage zu rechnen ist.

Der Sensor kann des Weiteren durch wenigstens einen optischen Sensor gebildet werden.

Dessen Sensorelement kann wenigstens eine Lichtquelle und wenigstens einen Empfänger aufweisen. Der Sensor kann Mittel zur Auswertung des von dem Empfänger erfassten Lichtes umfassen. So ist es beispielsweise denkbar, dass als Lichtquelle eine LED dient und dass als Empfänger ein Fotoelement dient, dass das von der Lichtquelle ausgehende Licht empfängt. Eine Änderung des Brechungsindex kann ausgewertet werden und auf das Vorhandensein von Flüssigkeit geschlossen werden.

In einer weiteren Ausgestaltung der Erfindung ist vorgesehen, dass das Sensorelement wenigstens einen Lichtleiter umfasst und, dass der Sensor Mittel zur Erfassung des durch den Lichtleiter gelangenden Lichtes umfasst. In diesem Fall besteht das Sensorelement somit aus einer oder mehreren optischen Fasern. Tritt eine Leckage auf und wird die Faser durch Flüssigkeit benetzt, kann sich die Lichtleitung ändern. Dies hat Einfluss auf die optische Signalübertragung, die ausgewertet werden kann, so dass dementsprechend auf die Anwesenheit von Flüssigkeit geschlossen werden kann.

Somit ist es denkbar, dass zur Detektion in einer Fläche ein Lichtleiter durch oder auf die fragliche Fläche gelegt wird. Dieser befindet sich zwischen einem Emitter und einem Empfänger des Sensors. Der Lichtleiter reflektiert einen hohen Anteil des Lichtes an seine Oberfläche. Befinden sich ein oder mehrere Tropfen auf den Lichtleiter, ändert sich der Brechungsindex und weniger Licht wird an den Seiten reflektiert. Dies hat zur Folge, dass der Empfänger weniger Licht detektiert. Dies kann ausgewertet werden und auf das Vorhandensein von Flüssigkeit geschlossen werden.

In einer weiteren Ausgestaltung der Erfindung ist vorgesehen, dass das Sensorelement derart ausgebildet ist, dass sich bei der Anwesenheit von Flüssigkeit die kapazitiven Eigenschaften des Sensorelementes ändern und, dass der Sensor Mittel zur Erfassung der kapazitiven Eigenschaften des Sensorelementes umfasst.

Somit können Sensorflächen so gestaltet werden, dass sich bei Flüssigkeitsaustritt aus einem Hydraulikkreislauf etc. die kapazitiven Eigenschaften von der Sensorfläche ändern. Dies ist bedingt durch die Änderung der Dielektrizitätszahl bei Anwesenheit von Flüssigkeit anstelle von Luft.

In einer weiteren Ausgestaltung der Erfindung ist vorgesehen, dass das Sensorelement derart ausgebildet ist, dass sich bei der Anwesenheit von Flüssigkeit die induktiven Eigenschaften des Sensorelementes ändern und dass der Sensor Mittel zur Erfassung der induktiven Eigenschaften des Sensorelementes umfasst. In diesem Fall muss sich die Sensorfläche in einem Magnetfeld befinden. Der erfassbare Messeffekt entsteht durch die erhöhten Wirbelstromverluste bei Anwesenheit von leitfähigen Medien anstelle von Luft im Magnetfeld. Diese Verluste können direkt gemessen werden. In einer alternativen Messmethode werden die Verluste kompensiert und dafür die eingebrachte Energie gemessen.

Gemäß der vorliegenden Erfindung ist vorgesehen, dass das Sensorelement bündig mit der Oberfläche des Gehäuses (bzw. in nicht beanspruchten Ausführungsformen mit der Oberfläche der Disposabelkassette) abschließt. Auch ist von nicht beanspruchten Ausführungsformen umfasst, dass das Sensorelement auf der Oberfläche des Gehäuses bzw. der Disposablekassette aufliegt oder auch unterhalb der Oberfläche angeordnet ist und die fragliche Eigenschaft durch die Oberfläche hindurch gemessen werden kann.

Die Vorteile der vorliegenden Erfindung bestehen darin, dass eine direkte Leckageüberwachung an flächigen Geometrien möglich ist, dass eine Leckageüberwachung an dreidimensionalen Formen möglich ist und dass eine großflächige Abdeckung ohne Weiteres realisierbar ist. Vor diesem Hintergrund kann die Anzahl von flüssigkeitsführenden Rinnen oder dergleichen, die die Flüssigkeit zum Sensorelement führen verringert werden bzw. diese können ganz weggelassen werden.

Durch die großflächige Abdeckung ist eine erhöhte Sicherheit gegeben und es werden weniger einzelne Sensoren benötigt. Vorzugsweise liegen geschlossene Oberflächen vor, d.h. keine Bohrungen für Flüssigkeitsführungen zum Sensor.

Gemäß der Erfindung ist das Sensorelement bzw. der Sensor in vorhandene Bauteile, nämlich in das Gehäuse des Gerätes (bzw. in nicht beanspruchten Ausführungsformen in die Oberfläche der Disposabelkassette) integriert.

Vorzugsweise ist das Sensorelement derart flexibel bzw. derart anpassbar, dass beliebige Überwachungsgeometrien und insbesondere dreidimensionale Geometrien denkbar sind.

Weitere Einzelheiten und Vorteile der Erfindung werden anhand eines in der Zeichnung dargestellten Ausführungsbeispiels näher erläutert. Es zeigen:
- Figur 1:: Eine schematische Schnittansicht durch die Oberfläche eines Gehäuses eines Dialysegerätes mit Sensor gemäß der Erfindung,
- Figur 2:: Eine mögliche Ausgestaltung des Sensorelementes in Form von Leiterbahnen,
- Figur 3:: Eine mögliche Ausgestaltung des Sensorelementes in Form einer Matrix und
- Figur 4:: Eine schematische Schnittansicht durch die Oberfläche eines Gehäuses eines Dialysegerätes mit Sensor gemäß dem Stand der Technik.

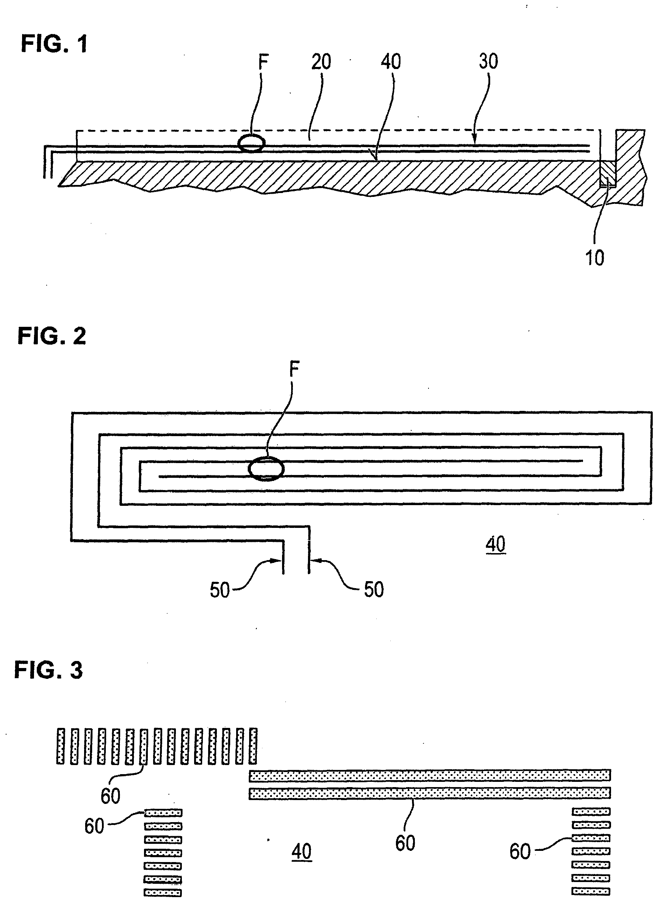

Figur 1 zeigt in einer Schnittansicht die Schnittstelle eines Dialysegerätes, an der ein nicht dargestelltes Disposable und insbesondere eine Disposablekassette angeordnet wird. Mit dem Bezugszeichen 30 ist ein Leckagesensor gekennzeichnet, der sich auf der Oberfläche 40 eines Dialysegerätes im Bereich der genannten Schnittstelle befindet. Zusätzlich dazu kann ein Sensor 10 gemäß dem Stand der Technik vorgesehen sein. Die Rinne 20 kann, muss jedoch nicht vorgesehen sein. Auch der Sensor 10 ist optional, da an sich die Detektion mittels des Flächensensors 30 ausreichend ist.

Der Flächensensor gemäß der Erfindung hat grundsätzlich vorzugsweise eine größere Erstreckung in Breiten- und Längenrichtung als in Dickenrichtung. Vorzugsweise übersteigen die Breite und/oder die Länge des Sensorelementes die Dicke des Sensorelementes gemäß der Erfindung um ein Vielfaches und vorzugsweise um den Faktor > 10.

Aufgrund der flächigen Ausgestaltung des Sensors 30 kann ein großer Bereich auf Leckage bzw. Feuchtigkeit hin überwacht werden. Dieser Bereich kann eine zweidimensionale Erstreckung oder auch eine dreidimensionale Erstreckung aufweisen. Wie oben ausgeführt, kann das Sensorelement bzw. der Sensor insgesamt so ausgebildet sein, dass eine resistive Messung, eine kapazitive Messung, eine induktive Messung oder beispielsweise auch eine optische Messung erfolgt.

Figur 2 zeigt ein Beispiel für die resistive Messung zwischen zwei Leiterbahnen 50, die das Sensorelement eines Sensors ausbilden. Wie dies aus Figur 2 ersichtlich ist, erstrecken sich die Leiterbahnen 50 auf dem Gehäuse des Dialysegerätes bzw. auf dessen Oberfläche 40.

Der elektrische Widerstand zwischen den beiden Leiterbahnen wird gemessen. Befindet sich ein Flüssigkeitstropfen F im Bereich zwischen zwei Leiterbahnen 50, ändert sich der Widerstand, was entsprechend festgestellt werden kann.

Anstelle von einer oder mehreren mäanderförmig angeordneten Leiterbahnen ist es auch denkbar, dass die elektrisch leitfähigen Elemente in Form einer Matrix angeordnet werden, deren Muster weitgehend beliebig ist und auf die jeweiligen Anforderungen hin zugeschnitten sein kann.

Ein solches Beispiel einer Matrix, das aus einzelnen elektrisch leitfähigen Elementen 60 besteht ist aus Figur 3 ersichtlich. Auch hier kann der elektrische Widerstand zwischen zwei oder mehr Elementen der Matrix gemessen werden. Dieser ändert sich bei Vorliegen von Flüssigkeit, die zwei oder mehr der dargestellten Elemente überbrückt. Dies kann festgestellt werden und somit auf das Vorhandensein einer Leckage geschlossen werden.

Bevorzugt ist es grundsätzlich, wenn der Abstand zwischen zwei leitfähigen Elementen, wie z.B. Leiterbahnen derart gewählt ist, dass ein auf der Oberfläche befindlicher Flüssigkeitstropfen wenigstens zwei der Leiterbahnen oder dergleichen tangiert oder überdeckt.

Grundsätzlich sind auch andere Sensorelemente wie beispielsweise optische Fasern denkbar, deren Lichtleitung sich bei Anwesenheit von Flüssigkeit bzw. Feuchtigkeit ändert. Dies kann dazu führen, dass ein durch einen Emitter ausgehendes Lichtsignal nicht den gesamten Lichtleiter durchdringt oder eine Signaländerung und insbesondere eine Signalabschwächung erfolgt.

Wie es beispielsweise aus Figur 2 hervorgeht ist das Sensorelement derart ausgebildet, dass es wesentlich größer ist als die Erstreckung eines Flüssigkeitstropfens, so dass die Wahrscheinlichkeit für die Erfassung von Flüssigkeit gegenüber einem lokal angeordneten Sensor, wie er aus Figur 4 ersichtlich ist, entsprechend erhöht ist.

## Patentansprüche

1. Dialysegerät mit wenigstens einem Gehäuse und mit wenigstens einem Sensor, der wenigstens ein Sensorelement aufweist, das zur Erfassung von Feuchtigkeit an der Oberfläche (40) des Gehäuses ausgebildet ist, wobei das Dialysegerät wenigstens eine Schnittstelle aufweist, an der ein Disposable angeordnet ist oder fixierbar ist, **dadurch gekennzeichnet, dass** sich das Sensorelement (50) über einen flächigen Bereich der Oberfläche des Gehäuses erstreckt und ein Teil des Gehäuses ist und bündig mit der Oberfläche des Gehäuses abschließt, und es sich bei der Oberfläche, an der sich das Sensorelement befindet, um eine Oberfläche der Schnittstelle handelt, so dass diese Oberfläche der Schnittstelle zu dem Disposable gleichzeitig das Gehäuse darstellt und als Sensor dient, wobei das Sensorelement wenigstens eine, vorzugsweise mehrere elektrisch leitende.Bahnen oder wenigstens eine elektrisch leitende Sensormatrix umfasst, und der Sensor des Weiteren Mittel zur Erfassung der Änderung des Widerstands oder der Leitfähigkeit der elektrisch leitenden Bahn oder der Sensormatrix umfasst.

2. Dialysegerät nach einem der vorhergehenden Ansprüche, **dadurch gekennzeichnet, dass** der flächige Bereich und das Sensorelement dreidimensional ausgebildet sind.

3. Dialysegerät nach einem der vorhergehenden Ansprüche, **dadurch gekennzeichnet, dass** keine Bohrung zur Aufnahme des Sensors oder des Sensorelementes und/oder keine Rinnen oder sonstige Bahnen zur Ableitung der Feuchtigkeit zu dem Sensor bzw. zu dem Sensorelement hin vorhanden sind.

4. Dialysegerät nach einem der vorhergehenden Ansprüche, **dadurch gekennzeichnet, dass** das Disposable eine Disposablekassette ist.

5. Dialysegerät nach einem der vorhergehenden Ansprüche, **dadurch gekennzeichnet, dass** das Sensorelement in Form von einer oder mehreren Bahnen oder in Form wenigstens einer Matrix ausgebildet ist.

6. Dialysegerät nach Anspruch 5, **dadurch gekennzeichnet, dass** die benachbarten Bahnen oder die benachbarten einzelnen Elemente der Matrix einen Abstand zueinander von 1 cm oder weniger haben.

7. Dialysegerät nach einem der vorhergehenden Ansprüche, **dadurch gekennzeichnet, dass** die elektrisch leitende Bahn oder die Sensormatrix durch ein Metall oder durch einen graphitgefüllten Kunststoff gebildet wird.

8. Dialysegerät nach einem der vorhergehenden Ansprüche, **dadurch gekennzeichnet, dass** das Sensorelement derart ausgebildet ist, dass sich bei der Anwesenheit von Flüssigkeit die kapazitiven Eigenschaften des Sensorelementes ändern und dass der Sensor Mittel zur Erfassung der kapazitiven Eigenschaften des Sensorelementes umfasst.

9. Dialysegerät nach einem der vorhergehenden Ansprüche, **dadurch gekennzeichnet, dass** das Sensorelement derart ausgebildet ist, dass sich bei der Anwesenheit von Flüssigkeit die induktiven Eigenschaften des Sensorelementes ändern und dass der Sensor Mittel zur Erfassung der induktiven Eigenschaften des Sensorelementes umfasst.

## Claims

1. A dialysis machine having at least one housing and having at least one sensor which has at least one sensor element which is configured for detecting moisture at the surface (40) of the housing, wherein the dialysis machine has at least one interface at which a disposable is arranged or can be fixed,
**characterized in that**
the sensor element (50) extends over an areal region of the surface of the housing and is a part of the housing and terminates flush with the surface of the housing and the surface at which the sensor element is located is a surface of the interface so that this surface of the interface with the disposable simultaneously represents the housing and serves as a sensor, with the sensor element comprising at least one electrically conductive track, preferably a plurality of electrically conductive tracks, or at least one electrically conductive sensor matrix, and the sensor furthermore comprising means for detecting the change in the resistance or in the conductivity of the electrically conductive track or of the sensor matrix.

2. A dialysis machine in accordance with one of the preceding claims, **characterized in that** the areal region and the sensor element are configured three-dimensionally.

3. A dialysis machine in accordance with one of the preceding claims, **characterized in that** no borehole is present for receiving the sensor or the sensor element and/or no channels or other tracks are present for conducting the moisture off toward the sensor or toward the sensor element.

4. A dialysis machine in accordance with one of the preceding claims, **characterized in that** the disposable is a disposable cassette.

5. A dialysis machine in accordance with one of the preceding claims, **characterized in that** the sensor element is configured in the form of one or more tracks or in the form of at least one matrix.

6. A dialysis machine in accordance with claim 5, **characterized in that** the adjacent tracks or the adjacent individual elements of the matrix have a spacing from one another of 1 cm or less.

7. A dialysis machine in accordance with one of the preceding claims, **characterized in that** the electrically conductive track or the sensor matrix is formed by a metal or by a graphite-filled plastic.

8. A dialysis machine in accordance with one of the preceding claims, **characterized in that** the sensor element is configured such that the capacitive properties of the sensor element change on the presence of fluid; and **in that** the sensor comprises means for detecting the capacitive properties of the sensor element.

9. A dialysis machine in accordance with one of the preceding claims, **characterized in that** the sensor element is configured such that the inductive properties of the sensor element change on the presence of fluid; and **in that** the sensor comprises means for detecting the inductive properties of the sensor element.

## Revendications

1. Appareil de dialyse comportant au moins un boîtier et au moins un capteur, qui présente au moins un élément de détection qui est conçu pour détecter de l'humidité sur la surface (40) du boîtier, l'appareil de dialyse présentant au moins une interface sur laquelle un article à usage unique est disposé ou peut être fixé,
**caractérisé en ce que** l'élément de détection (50) s'étend sur une zone plane de la surface du boîtier et fait partie du boîtier et se termine au même niveau que la surface du boîtier, et la surface sur laquelle se trouve l'élément de détection est une surface de l'interface, de telle sorte que cette surface de l'interface avec l'article à usage unique représente le boîtier et en même temps sert de capteur, l'élément de détection comprenant au moins une, de préférence plusieurs bandes électriquement conductrices ou au moins une matrice de détection électriquement conductrice, et le capteur comprenant en outre des moyens pour détecter le changement de la résistance ou de la conductivité de la piste électriquement conductrice ou de la matrice de détection.

2. Appareil de dialyse selon l'une des revendications précédentes, **caractérisé en ce que** la zone plane et l'élément de détection sont réalisés en trois dimensions.

3. Appareil de dialyse selon l'une des revendications précédentes, **caractérisé en ce qu'**il n'y a aucun trou pour recevoir le capteur ou l'élément de détection et/ou aucune rainure ou autre voie de déviation de l'humidité vers le capteur ou vers l'élément de détection.

4. Appareil de dialyse selon l'une des revendications précédentes, **caractérisé en ce que** l'article à usage unique est une cassette à usage unique.

5. Appareil de dialyse selon l'une des revendications précédentes, **caractérisé en ce que** l'élément de détection est réalisé sous la forme d'une ou de plusieurs pistes ou sous la forme d'au moins une matrice.

6. Appareil de dialyse selon la revendication 5, **caractérisé en ce que** les pistes adjacentes ou les différents éléments adjacents de la matrice sont espacés les uns des autres de 1 cm ou moins.

7. Appareil de dialyse selon l'une des revendications précédentes, **caractérisé en ce que** la piste électriquement conductrice ou la matrice de détection est formée par un métal ou par une matière plastique remplie de graphite.

8. Appareil de dialyse selon l'une des revendications précédentes, **caractérisé en ce que** l'élément de détection est conçu de telle manière que, en présence de liquide, les propriétés capacitives de l'élément de détection se modifient et **en ce que** le capteur comprend des moyens pour détecter les propriétés capacitives de l'élément de détection.

9. Appareil de dialyse selon l'une des revendications précédentes, **caractérisé en ce que** l'élément de détection est conçu de telle manière que, en présence de liquide, les propriétés inductives de l'élément de détection se modifient et **en ce que** le capteur comprend des moyens pour détecter les propriétés inductives de l'élément de détection.
